(19) Europäisches Patentamt
European Patent Office
Office européen des brevets

(11) Veröffentlichungsnummer : **0 127 797**
**B1**

(12) **EUROPÄISCHE PATENTSCHRIFT**

(45) Veröffentlichungstag der Patentschrift : **16.06.87**

(51) Int. Cl.⁴ : **C 07 D487/22**, C 09 B 47/00, G 01 N 33/533

(21) Anmeldenummer : **84105272.3**

(22) Anmeldetag : **10.05.84**

(54) **Markermoleküle für Fluoreszenz-Immuno-Assays sowie Verfahren und Zwischenprodukte zu deren Herstellung.**

(30) Priorität : **03.06.83 CH 3045/83**

(43) Veröffentlichungstag der Anmeldung : **12.12.84 Patentblatt 84/50**

(45) Bekanntmachung des Hinweises auf die Patenterteilung : **16.06.87 Patentblatt 87/25**

(84) Benannte Vertragsstaaten : **BE CH DE FR GB IT LI NL**

(56) Entgegenhaltungen :
**EP-A- 0 071 991**
**CH-A- 596 211**
**PATENT ABSTRACTS OF JAPAN, unexamined applications, C Sektion, Vol. 3, No. 118, 4. Oktober 1979, THE PATENT OFFICE JAPANESE GOVERNMENT. Seite 141 C 60**
**PATENT ABSTRACTS OF JAPAN, unexamined applications, C Field, Vol. 4, No. 140, 3. Oktober 1980, THE PATENT OFFICE JAPANESE GOVERNMENT, Seite 3 C 26**
**PATENT ABSTRACTS OF JAPAN, unexamined applications, C Field, Vol. 7, No. 277, 9. Dezember 1983, THE PATENT OFFICE JAPANESE GOVERNMENT, Seite 8 C 199**

(73) Patentinhaber : **F. HOFFMANN-LA ROCHE & CO. Aktiengesellschaft**
**CH-4002 Basel (CH)**

(72) Erfinder : **Schmidt, Dieter, Dr.**
**Redingstrasse 20**
**CH-4052 Basel (CH)**
Erfinder : **Steffen, Hans, Dr.**
**Olsbergerstrasse 27**
**CH-4411 Arisdorf (CH)**

(74) Vertreter : **Kloter, Rudolf et al**
**Postfach 3255 Grenzacherstrasse 124**
**CH-4002 Basel (CH)**

**EP 0 127 797 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

In der JP-A-54-100 398 und der JP-A-55-87 793 werden Mesotetra (p-sulphophenyl) porphinderivate bzw. Meso-tetra (p-carboxyphenyl) porphinderivate beschrieben, welche als anticarcinogene Mittel bzw. als Zwischenprodukte zu deren Herstellung dienen sollen.

Demgegenüber betrifft die vorliegende Erfindung Porphyrinderivate, die sich fluoreszenzspektroskopisch sehr empfindlich nachweisen lassen und, da sie wasserlöslich sind, bestens geeignet sind als Markermoleküle für hochempfindliche Fluoreszenz-Immunoassays.

Die Porphyrinderivate gemäss vorliegender Erfindung entsprechen der allgemeinen Formel

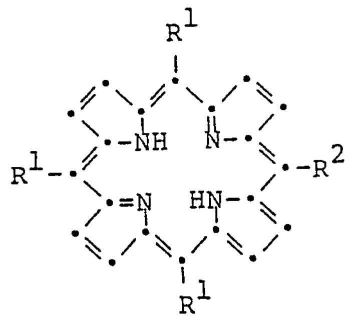

(I)

worin entweder $R^1$ die Gruppe

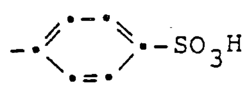

und $R^2$ die Gruppe

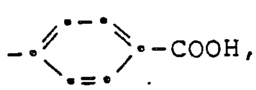,
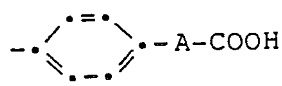

oder

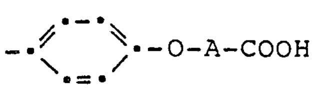

oder $R^1$ die Gruppe

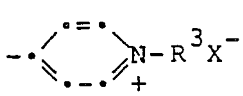

und $R^2$ die Gruppe

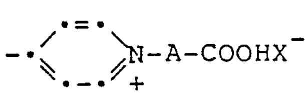

bedeuten, wobei $X^-$ ein Chlor-, Brom- oder Jodanion, ein Arylsulfonation, ein Alkylsulfonation oder ein Alkylsulfation, A $(C_{1-8})$-Alkylen und $R^3$ $(C_{1-4})$-Alkyl bedeuten, und Sulfonsäuresalze von Verbindungen der Formel I, worin $R^1$ die Gruppe

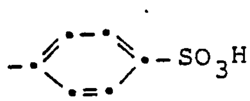

bedeutet.

Die vorliegende Erfindung betrifft ferner ein Verfahren und Zwischenprodukte für die Herstellung dieser Verbindungen.

Die Ausdrücke « Alkyl » und « Alkylen » bezeichnen geradkettige oder verzweigte, gesättigte Kohlenwasserstoffreste. Der Ausdruck « $(C_{1-4})$-Alkyl » bezeichnet Reste mit 1 bis 4 Kohlenstoffatomen,

wie Methyl und dergleichen. Der Ausdruck « $(C_{1-8})$-Alkylen » bezeichnet Reste mit 1 bis 8 Kohlenstoffatomen, wie Methylen, Pentamethylen und dergleichen.

Im Rahmen der vorliegenden Erfindung bevorzugte Verbindungen der Formel I sind :

4-[10,15,20-Tris(4-sulfophenyl)-21H,23H-porphin-5-yl]benzoesäure,

[4-[10,15,20-Tris(4-sulfophenyl)-21H,23H-porphin-5-yl]phenoxy]essigsäure,

6-[4-[10,15,20-Tris(4-sulfophenyl)-21H,23H-porphin-5-yl]phenyl]hexancarbonsäure,

1-(Carboxymethyl)-1',1'',1'''-trimethyl-4,4',4'',4'''-(21H,23H-porphin-5,10,15,20-tetrayl)tetrakispyridinium tetrajodid,

1-(2-Carboxyäthyl)-1',1'',1'''-trimethyl-4,4',4'',4'''-(21H,23H-porphin-5,10,15,20-tetrayl)tetrakispyridinium. bromid trijodid und

1-(5-Carboxypentyl)-1',1'',1'''-trimethyl-4,4',4'',4'''-(21H,23H-porphin-5,10,15,20-tetrayl)tetrakispyridinium bromid trijodid.

Die Verbindungen der Formel I und, wenn $R^1$ die Gruppe

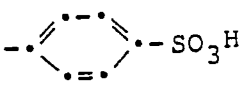

bedeutet, die entsprechenden Sulfonsäuresalze können erfindungsgemäss hergestellt werden, indem man

a) eine Verbindung der allgemeinen Formel

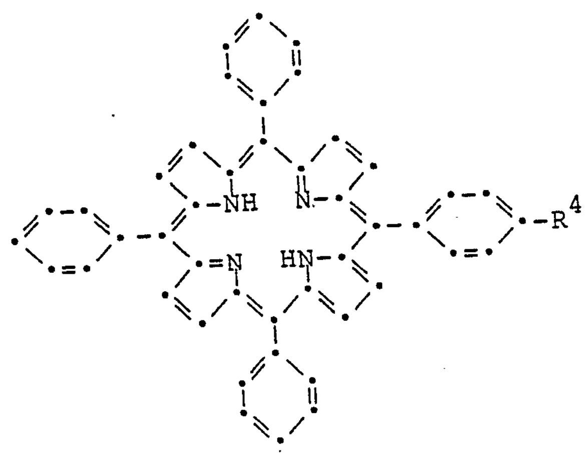

(II)

worin $R^4$ die Gruppe —COOH, —A—COOH oder —O—A—COOH und A $(C_{1-8})$-Alkylen bedeuten, mit einem die Gruppe —$SO_3H$ liefernden Mittel umsetzt, oder

b) eine Verbindung der allgemeinen Formel

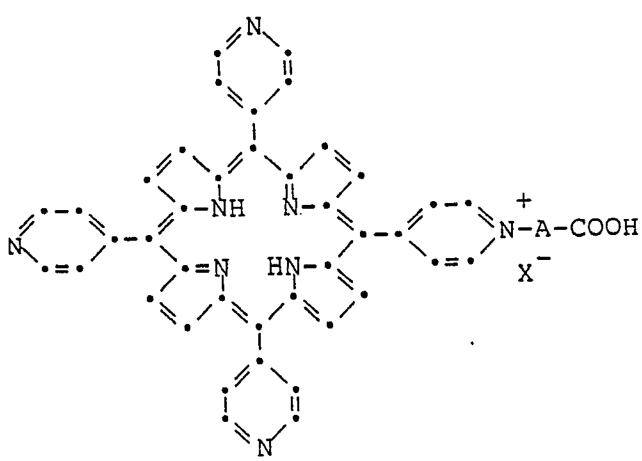

(III)

worin A $(C_{1-8})$-Alkylen und $X^-$ ein Chlor-, Brom- oder Jodanion, ein Arylsulfonation, ein Alkylsulfonation oder ein Alkylsulfation bedeuten, mit einem einen $(C_{1-4})$-Alkylrest liefernden Mittel behandelt und erwünschtenfalls

c) eine nach Verfahren a) erhaltene Verbindung in ein Sulfonsäuresalz überführt.

Gemäss Verfahrensvariante a) können Verbindungen der Formel I, worin $R^1$ die Gruppe

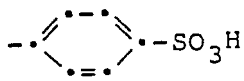

und $R^2$ die Gruppe

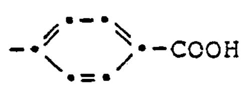

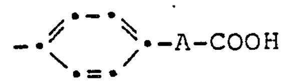

oder

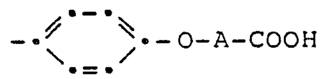

bedeuten, durch Sulfonierung der Phenylgruppen in einer Verbindung der Formel II hergestellt werden. Als Sulfonierungsmittel verwendet man vorzugsweise konzentrierte Schwefelsäure, wobei man bei erhöhter Temperatur, z. B. bei etwa 100 °C, arbeitet. Die gewünschte Sulfonierung kann jedoch auch nach anderen an sich bekannten Methoden durchgeführt werden.

Gemäss Verfahrensvariante b) können Verbindungen der Formel I, worin $R^1$ die Gruppe

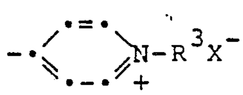

und $R^2$ die Gruppe

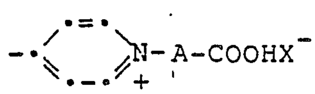

bedeuten, durch Alkylierung der Pyridinstickstoffatome in einer Verbindung der Formel III hergestellt werden. Als Alkylierungsmittel können entsprechende Alkylchloride, -bromide oder -jodide, wie Methyljodid, Arylsulfonsäure-Alkylester, wie Aethyl p-Toluolsulfonat, Alkylsulfonsäure-Alkylester, Dialkylsulfate, wie Diäthylsulfat, und dergleichen verwendet werden. Geeignete Lösungsmittel sind in erster Linie polare Lösungsmittel, beispielsweise Alkohole, wie Methanol, und Mischungen davon mit Wasser. Die Reaktion wird vorzugsweise bei Raumtemperatur durchgeführt.

Die Herstellung der Sulfonsäuresalze gemäss Verfahrensvariante c) erfolgt nach an sich bekannten und jedem Fachmann geläufigen Methoden. Es kommen dabei sowohl Salze mit anorganischen Basen, z. B. Alkalimetallsalze, als auch Salze mit organischen Basen, z. B. Ammoniumsalze, in Frage. Bevorzugt werden die entsprechenden Natriumsalze.

Die als Ausgangsstoffe verwendeten Verbindungen der Formel II sind neu und ebenfalls Gegenstand der vorliegenden Erfindung. Sie können hergestellt werden, indem man Pyrrol, Benzaldehyd und einen Aldehyd der allgemeinen Formel

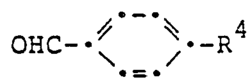

(IV)

worin $R^4$ die obige Bedeutung besitzt, im Molverhältnis 4 : 3 : 1 miteinander kondensiert. Diese Kondensation erfolgt nach an sich bekannten und jedem Fachmann geläufigen Methoden. In einer bevorzugten Ausführungsform wird das Pyrrol in einem sauren organischen Lösungsmittel, wie Essigsäure und Propionsäure, vorgelegt, und ein Gemisch der beiden. Aldehyde (vorzugsweise im Molverhältnis 3 : 1) langsam zugegeben. Die Reaktionstemperatur ist zwar nicht kritisch, man arbeitet jedoch vorzugsweise bei erhöhter Temperatur, beispielsweise bei der Siedetemperatur der Reaktionsmischung. Die Auftrennung des Produktgemisches erfolgt zweckmässigerweise in an sich bekannter Weise mittels chromatographischer Methoden.

Die Verbindungen der Formel IV sind bekannt oder können nach an sich bekannten Methoden und in Analogie zur Herstellung bekannter Vertreter dieser Stoffklasse hergestellt werden.

Die als Ausgangsstoffe verwendeten Verbindungen der Formel III sind neu und ebenfalls Gegenstand der vorliegenden Erfindung. Sie können hergestellt werden, indem man die Verbindung der Formel

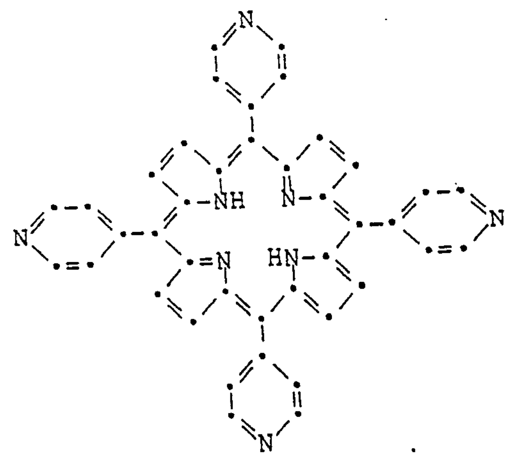

(V)

mit einer Verbindung der allgemeinen Formel

X—A—COOH VI

worin X Chlor, Brom, Jod oder einen Alkylsulfonat-, Alkylsulfat oder einen Arylsulfonatrest bedeutet und A die obige Bedeutung besitzt, umsetzt. Als Lösungsmittel eignen sich beispielsweise halogenierte Kohlenwasserstoffe, wie Methylenchlorid und Chloroform, Alkohole, wie Methanol und Aethanol, Mischungen der erwähnten Lösungsmittel und dergleichen. Je nach Reaktivität der eingesetzten Verbindung der Formel VI kann die Reaktion in einem Temperaturbereich von etwa Raumtemperatur bis zur Siedetemperatur der Reaktionsmischung durchgeführt werden.

Die Verbindung der Formel V kann durch Kondensation von Pyrrol und Pyridin-4-carbaldehyd (Molverhältnis 1 : 1) nach hinlänglich bekannten Methoden hergestellt werden. Es können beispielsweise die gleichen Reaktionsbedingungen wie bei der früher beschriebenen Herstellung von Verbindungen der Formel II angewendet werden.

Die Porphyrine gemäss allgemeiner Formel I bzw. deren Sulfonsäuresalze lassen sich fluoreszenzspetroskopisch sehr empfindlich nachweisen. Da sie wasserlöslich sind, sind sie bestens geeignet als Markermoleküle für hochempfindliche Fluoreszenz-Immuno-Assays. Sie sind insbesondere geeignet für einen zeitaufgelösten Fluoreszenz-Immuno-Assay, wie er beispielsweise in der DT-OS 26 28 158 beschrieben wird. Durch die Verwendung der Porphyrine gemäss allgemeiner Formel I anstelle des häufig verwendeten FITC (Fluoresceinisocyanat) kann die Nachweisempfindlichkeit bei Fluoreszens-Immuno-Assays verbessert werden. Dies ist insbesondere bei der Bestimmung geringer Mengen von Antigenen in Körperflüssigkeiten, wie z. B. Plasma und Serum, von Vorteil. Ein Beispiel eines solchen Antigens ist das carcinoembryonale Antigen (CEA). Die Kopplung einer Verbindung der allgemeinen Formel I an ein immunologisches Material erfolgt in herkömmlicher Weise, z. B. mit einem wasserlöslichen Carbodiimid-Derivat, z. B. mit 1-Cyclohexyl-3-(2-morpholinoäthyl)-carbodiimid-p-toluolsulfonat.

## Beispiel 1

a) Man erhitzt eine Mischung aus 2,5 ml Pyrrol und 134 ml Propionsäure zum Sieden und versetzt dann tropfenweise mit einer Lösung von 2 g p-Formylphenylcapronsäure und 3 ml Benzaldehyd in 5 ml Propionsäure. Man erhitzt noch während 30 Minuten unter Rückfluss zum Sieden, lässt die Reaktionslösung auf Zimmertemperatur abkühlen und neutralisiert die Propionsäure mit insgesamt 9,5 g Natriumhydroxid (pH 4). Man lässt über Nacht stehen und filtriert dann den schwarzen Niederschlag ab. Die Reinigung erfolgt durch Säulenchromatographie an 1,2 kg Kieselgel, wobei man nacheinander mit den folgenden Lösungsmitteln eluiert : Chloroform/Cyclohexan (1 : 1), Chloroform/Essigester (3 : 2) und (2 : 3), Essigester und Essigester mit 2 % Methanol. Das so erhaltene Produkt wird noch zweimal wie oben beschrieben chromatographiert und dann umkristallisiert. Hierzu wird das Produkt im möglichst wenig Chloroform gelöst, worauf man die doppelte Menge an Methanol zugibt und über Nacht bei 4 °C stehen lässt. Nach dem Abfiltrieren werden die Kristalle mit Methanol gewaschen und dann im Vakuum bei 20 °C getrocknet. Man erhält 810 mg (12 %) 6-[4-(10,15,20-Triphenyl-21H,23H-porphin-5-yl)phenyl]hexancarbonsäure.

b) Man suspendiert 400 mg fein pulverisierte 6-[4-(10,15,20-Triphenyl-21H,23H-porphin-5-yl))phenyl]hexancarbonsäure in 10 ml konzentrierter Schwefelsäure, erhitzt dieses Gemisch unter Feuchtigkeits- und Lichtausschluss während 6 Stunden auf 100 °C und lässt dann über Nacht bei 20 °C stehen. Anschliessend gibt man vorsichtig 15 ml Wasser hinzu, wobei sich das Gemisch erwärmt, und lässt wieder auf Zimmertemperatur abkühlen. Das ausgefallene grüne protonierte Produkt wird abfiltriert, mit wenig Aceton gewaschen, zusammen mit etwas Celite TM in 15 ml Wasser suspendiert und mit gesättigter Natriumbicarbonatlösung neutralisiert, bis sich die Lösung von grün nach rot-violett verfärbt. Reste von

nicht umgesetztem Ausgangsprodukt werden zusammen mit dem Celite TM abfiltriert. Das Filtrat (etwa 100 ml) wird 4 mal je 3 Stunden lang gegen 4 Liter Wasser dialysiert (um anorganische Salze zu entfernen) und dann lyophilisiert. Die Reinigung erfolgt durch Chromatographie an Acrylamid-Gel AcA54 (LKB) ; Elutionsmittel : 150 mM Kochsalzlösung, das 10 mMol/l Natriumphosphat enthält (pH 7). Die Fraktionen mit reinem Produkt werden gepoolt und zur Entfernung des Kochsalzes gegen Wasser dialysiert und dann lyophilisiert. Man erhält 189 mg (35 %) 6-[4-[10,15,20-Tris(4-sulfophenyl)-21H,23H-porphin-5-yl]phenyl]hexancarbonsäure-tetranatriumsalz.

UV : λ (ε), 414 (364 000), 516 (12 700), 553 (6 100), 578 (5 600), 634 (3 400) nm.

## Beispiel 2

a) In Analogie zu den Angaben in Beispiel 1 a) erhält man aus Pyrrol, Benzaldehyd und p-Formylphenoxyessigsäure in einer Ausbeute von 7 % [4-(10,15,20-Triphenyl-21H,23H-porphin-5-yl)phenoxy]essigsäure.

Die als Ausgangsstoff verwendete p-Formyl-phenoxyessigsäure wird aus p-Hydroxybenzaldehyd und Jodessigsäure in an sich bekannter Weise hergestellt.

b) In Analogie zu den Angaben in Beispiel 1 b) erhält man aus [4-(10,15,20-Triphenyl-21H,23H-porphin-5-yl)-phenoxy]essigsäure in einer Ausbeute von 15 % [4-[10,15,20-Tris(4-sulfophenyl)-21H,23H-porphin-5-yl]phenoxy]essigsäure.

UV : λ (ε), 414 (397 000), 517 (13 000), 554 (6 400), 579 (5 600), 635 (3 100) nm.

## Beispiel 3

a) Man erhitzt 187 ml Propionsäure unter Rühren am Rückfluss und versetzt dann zuerst mit 3,47 ml Pyrrol und dann tropfenweise (innert 15 Minuten) mit 4,81 ml Pyridin-4-carbaldehyd. Man erhitzt während 30 Minuten am Rückfluss, lässt die Reaktionslösung auf Zimmertemperatur abkühlen, versetzt mit 105 g Natriumhydroxid in 400 ml Wasser, wobei mit Eis gekühlt wird, und nutscht den erhaltenen schwarzen Niederschlag ab. Zur Reinigung löst man diesen in 400 ml Chloroform und fällt das Produkt dann durch langsame Zugabe von 700 ml Cyclohexan aus. Durch dreimaliges Aufschlämmen in wenig Chloroform und anschliessendem Abnutschen werden noch weitere Verunreinigungen entfernt. Die weitere Reinigung erfolgt durch zweimalige Säulenchromatographie an Kieselgel ; Elutionsmittel : Chloroform, Chloroform/Methanol (9 : 1), Chloroform/Methanol (8 : 2) und Chloroform/Methanol (7 : 3). Das erhaltene Material wird in Aceton aufgeschlämmt, abgenutscht und an Kieselgel unter Eluieren mit Chloroform, das 3 % Methanol enthält, chromatographiert. Man erhält 600 mg (8 %) 5,10,15,20-Tetra(4-pyridyl)-21H,23H-porphin.

b) Man löst 180 mg 5,10,15,20-Tetra(4-pyridyl)-21H,23H-porphin in 70 ml Chloroform/Methanol (9 : 1), versetzt mit 585 mg 6-Brom-Capronsäure und erhitzt anschliessend während 20 Stunden am Rückfluss. Dann engt man die Lösung im Vakuum auf 5 bis 10 ml ein und fällt das Produkt durch Zugabe von 250 ml Aether aus. Dieses wird auf eine 15 cm langen Kieselsäule gegeben, worauf man mit 500 ml Chloroform/Methanol (9 : 1) eluiert, die Startzone aus der Säule nimmt und diese mit 1 Liter Methanol und dann mit 1 Liter Chloroform/Methanol/Wasser (2 : 7 : 1) extrahiert. Das aus den Extrakten erhaltene Material wird durch präparative Dickschichtchromatographie weiter gereinigt ; Laufmittel Chloroform/Methanol/Dimethylformamid/Wasser (1 : 2 : 1 : 1). Die Hauptzone wird nach dem Herauskratzen zweimal mit je 250 ml Methanol und zweimal mit je 250 ml Chloroform/Methanol/Wasser (2 : 7 : 1) extrahiert. Schliesslich wird das Produkt noch viermal umgefällt. Hierzu löst man es zunächst in möglichst wenig Methanol und fällt es dann durch langsames Zutropfen von 250 ml Chloroform aus. Man erhält 30 mg 1-(5-Carboxypentyl)-4-(10,15,20-tri-4-pyridyl-21H,23H-porphin-5-yl)pyridinium bromid.

c) Man löst 20 mg 1-(5-Carboxypentyl)-4-(10,15,20-tri-4-pyridyl-21H,23H-porphin-5-yl)pyridinium bromid in 50 ml Methanol/Wasser (9 : 1), gibt 3,5 g Methyljodid dazu und lässt das Reaktionsgemisch bei Zimmertemperatur während 6 Tagen im Dunkeln stehen. Dann wird das Lösungsmittel mit dem überschüssigen Methyljodid im Vakuum abgezogen und das Produkt durch Umfällen gereinigt. Hierzu löst man es in möglichst wenig Methanol (etwa 5 ml) und fällt es durch langsames Zutropfen von 250 ml Chloroform wieder aus. Diese Reinigungsprozedur wird insgesamt dreimal durchgeführt. Man erhält 7 mg 1-(5-Carboxypentyl)-1′,1″, 1‴-trimethyl-4,4′,4″,4‴-(21H,23H-porphin-5,10,15,20-tetrayl)tetrakispyridinium bromid trijodid.

UV : λ (ε), 422 (224 000), 519 (14 300), 556 (6 200), 584 (6 800), 640 (2 000) nm.

## Beispiel 4

a) Man löst 15,2 ml Benzaldehyd und 7,51 g 4-Carboxybenzaldehyd in 746 ml siedender Propionsäure, versetzt tropfenweise mit 13,9 ml Pyrrol, erhitzt anschliessend das erhaltene Gemisch während etwa 30 Minuten unter Rückfluss und lässt dann auf Zimmertemperatur abkühlen. Das Porphinderivat wird

durch Zugabe von 0,5 l Wasser ausgefällt. Das abfiltrierte Rohprodukt wird durch zweimalige Säulenchromatographie an 1,2 kg Kieselgel vorgereinigt, wobei jeweils nacheinander mit den folgenden Lösungsmitteln eluiert wird : Chloroform/Cyclohexan (1 : 1), Chloroform/Essigester (3 : 2) und (1 : 1), Essigester sowie Essigester, das 2 % Methanol enthält. Zur weiteren Reinigung schlämmt man das Produkt in wenig Methanol auf und filtriert es ab. Dieser Prozess wird so lange wiederholt bis das Filtrat nur noch schwach gefärbt ist. Dann wird das so erhaltene Produkt an einer Kieselgelsäule mit Chloroform, Chloroform/Essigester (1 : 1) und Essigester als Elutionsmittel chromatographiert. Man erhält 1,7 g 4-(10,15,20-Triphenyl-21H,23H-porphin-5-yl)benzoesäure als violette Kristalle.

b) In Analogie zu den Angaben in Beispiel 1 b) erhält man aus 200 mg 4-(10,15,20-Triphenyl-21H,23H-porphin-5-yl)benzoesäure 90 mg (33 %) reines 4-[10,15,20-Tris(4-sulfophenyl)-21H,23H-porphin-5-yl]benzoesäure-tetranatriumsalz.

UV : λ (ε), 413 (418 000), 517 (13 600), 554 (6 900), 578 (5 900), 634 (3 200) nm.

## Beispiel 5

a) Man löst 430 mg Tetrapyridylporphin in 150 ml Chloroform/Methanol (9 : 1), versetzt mit 1,05 g 3-Brom-propionsäure und erhitzt das Gemisch unter Rühren 18 Stunden lang auf 60 °C. Dann engt man die Lösung im Vakuum auf etwa 10 ml ein und fällt das Produkt durch langsame Zugabe von 300 ml Aether aus. Das abfiltrierte Porphingemisch wird auf eine Kieselgelsäule (Länge 15 cm, Durchmesser 2 cm) gegeben, worauf man mit 500 ml Chloroform/Methanol (9 : 1) eluiert. Dabei bleibt das gewünschte Produkt als violette Zone am Start zurück. Diese Startzone wird aus der Säule genommen und mit 2 l Methanol, sowie mit 2 l Chloroform/Methanol/Dimethylformamid/Wasser (1 : 2 : 1 : 1) extrahiert. Die weitere Reinigung des Produktes erfolgt mittels präparativer Dickschichtchromatographie «Laufmittel : Chloroform/Methanol/Dimethylformamid/Wasser (1 : 2 : 1 : 1)]. Die Hauptzone wird nach dem Herauskratzen viermal mit je 250 ml Chloroform/Methanol/Wasser (2 : 7 : 1) extrahiert. Schliesslich wird das Produkt noch viermal umgefällt, indem man es in möglichst wenig (~ 40 ml) Chloroform/Methanol/Wasser (2 : 7 : 1) löst und dann durch langsames Zutropfen von 300 ml Chloroform wieder ausfällt. Man erhält 42 mg reines 1-(2-Carboxyäthyl)-4-(10,15,20-tri-4-pyridyl-21H,23H-porphin-5-yl)-pyridinium bromid als violette Kristalle.

b) Man löst 40 mg 1-(2-Carboxyäthyl)-4-(10,15,20-tri-4-pyridyl-21H,23H-porphin-5-yl)-pyridinium bromid in 120 ml Chloroform/Methanol/Wasser (2 : 7 : 1), gibt 7,1 g Methyljodid dazu und lässt das Reaktionsgemisch bei Zimmertemperatur während 6 Tagen im Dunkeln stehen. Dann wird das Volumen der Lösung im Vakuum auf 20 ml eingeengt, und das Produkt durch langsame Zugabe von 20 ml Aether ausgefällt. Nach dem Abfiltrieren und Auswaschen des Produktes mit Aether wird dieses durch dreimaliges Umfällen gereinigt. Hierzu löst man es jeweils in 20 ml Methanol und fällt es durch langsames Zutropfen von 200 ml Aether wieder aus. Man erhält 18,5 mg reines 1-(2-Carboxyäthyl)-1',1'',1'''-trimethyl-4,4',4'',4'''-(21H,23H-porphin-5,10,15,20-tetrayl)tetrakispyridinium bromid trijodid als violettes Pulver.

UV : λ (ε), 422 (236 000), 519 (14 600), 554 (5 700), 584 (6 200), 640 (1 400) nm.

## Beispiel 6

a) Man löst 619 mg Tetrapyridylporphin und 2,79 g Jodessigsäure in 300 ml Chloroform/Methanol (9 : 1) auf und lässt dieses Gemisch 22 Stunden bei Zimmertemperatur im Dunkeln stehen. Dann wird die Lösung im Vakuum auf etwa 20 ml eingeengt, und das Produkt durch langsames Zutropfen von 400 ml Aether ausgefällt. Das abfiltrierte und gut mit Aether ausgewaschene Porphingemisch wird auf eine Kieselgelsäule (Länge 15 cm, Durchmesser 2 cm) gegeben, worauf man mit 500 ml Chloroform/Methanol (9 : 1) eluiert. Das gewünschte Produkt bleibt dabei als violette Startzone zurück. Diese wird aus der Säule genommen und mit Chloroform/Methanol/Wasser (1 : 3,5 : 0,5) extrahiert. Die weitere Reinigung erfolgt mittels präparativer Dickschichtchromatographie (vgl. Beispiel 5), sowie durch Chromatographie an einer Kieselgelsäule [Länge 20 cm, Durchmesser 1 cm, Laufmittel : Chloroform/Methanol/Wasser (2 : 7 : 1)]. Schliesslich wird das Produkt noch insgesamt dreimal umgefällt, indem man es in etwa 30 ml Chloroform/Methanol/Wasser (2 : 7 : 1) löst und dann durch langsames Zutropfen von 300 ml Aether wieder ausfällt. Man erhält 68 mg 1-(Carboxymethyl)-4-(10,15,20-tri-4-pyridyl-21H,23H-porphin-5-yl)pyridinium jodid als violettes Pulver.

b) Man löst 68 mg 1-(Carboxymethyl)-4-(10,15,20-tri-4-pyridyl-21H,23H-porphin-5-yl)pyridinium jodid in 350 ml Chloroform/Methanol/Wasser (2 : 7 : 1), gibt 14 g Methyljodid dazu und lässt das Reaktionsgemisch bei Zimmertemperatur während 6 Tagen im Dunkeln stehen. Dann wird im Vakuum auf etwa 40 ml eingeengt, mit 40 ml Methanol versetzt, und das Produkt durch langsame Zugabe von 350 ml Aether ausgefällt. Nach dem Abfiltrieren und Auswaschen des Produktes mit Aether wird dieses durch dreimaliges Umfällen gereinigt. Hierzu löst man das Produkt jeweils in 40 ml Methanol und fällt es durch langsames Zutropfen von 300 ml Aether wieder aus. Man erhält 36 mg 1-(Carboxymethyl)-1',1'',1'''-trimethyl-4,4',4'',4'''-(21H,23H-porphin-5,10,15,20-tetrayl)tetrakispyridinium tetrajodid.

UV : λ (ε), 422 (210 000), 519 (14 100), 554 (6 000), 584 (6 200), 640 (1 600) nm.

Beispiel 7

Markierung von anti-CEA mit 6-[4-[10,15,20-Tris(4-sulfophenyl)-21H,23H-porphin-5-yl]phenyl]hexancarbonsäure tetranatriumsalz.

Die Kopplung des obenerwähnten Porphinderivates an anti-CEA erfolgte wie unten beschrieben mit Hilfe des wasserlöslichen Carbodiimid-Derivates 1-Cyclohexyl-3-(2-morpholinäthyl)-carbodiimid-methyl-p-toluol-sulfonat.

Für die Kopplungsreaktion stellt man die folgenden Stammlösungen her :

1. 4 mg/ml Porphinderivat in Wasser bei pH 4,5 ; olivgrüne Lösung.

2. 2,9 mg/ml anti-CEA vom Kaninchen (DAKO Code No. A 115, Lot 042 A) in 200 mM $NaHCO_3$ ; pH 8.6.

Zu 3,2 mg 1-Cyclohexyl-3-(2-morpholinoäthyl)-carbodiimid-methyl-p-toluolsulfonat gibt man 400 μl der Stammlösung 1 mit dem Porphinderivat und durchmischt kurz am Vortex. Nach 2 minuten fügt man 400 μl der anti-CEA-Stammlösung 2 hinzu und durchmischt kurz am Vortex. Die Lösung verfärbt sich dabei rot und weist einen pH von 8,2 auf. Dieser wird mit wenig 1 N NaOH auf 8,6 eingestellt. Dann lässt man das Reaktionsgemisch im Dunkeln 16 Stunden bei Zimmertemperatur stehen.

Zur Abtrennung des markierten anti-CEA's wurden 500 μl des Reaktionsgemisches über eine Säule (Länge 30 cm, Durchmesser 9 mm) mit Acrylamid Gel (AcA-54 von LKB) chromatographiert (Eluiermittel : 150 mM Natriumchlorid, 10 mM Natriumphosphat, 0,02 % Natriumazid, pH 7,0). Die Fraktionen mit dem höchsten Gehalt an markiertem anti-CEA wurden gepoolt — insgesamt 5 ml. In dieser Lösung wurde UVspektroskopisch der Gehalt an anti-CEA (bei 278 nm) und an Porphyrinderivat (bei 417 nm) bestimmt. Es wurden dabei folgende Konzentrationen erhalten :

$0{,}31 \times 10^{-6}$ M/l Porphinderivat und
$0{,}73 \times 10^{-6}$ M/l anti-CEA.
Dies entspricht einem Markierungsgrad von 0,42.

Beispiel 8

Durchführung eines Fluoreszenzimmunoassays

Quantitative Bestimmung von CEA-Standards mit einem monoklonalen CEA-Antikörper und einem gebräuchlichen CEA-Antikörper (Kaninchen) :

In die erforderliche Anzahl Teströhrchen (10 × 75 mm) werden je 0,250 ml CEA-Standardlösung (0 ng/ml CEA ; 2,0 ng/ml CEA ; 5 ng/ml CEA ; 10 ng/ml CEA und 20 ng/ml CEA in 0,2 M/l Natriumacetat, pH 5 mit 4 g/l Rinderserumalbumin) pipettiert, je eine mit monoklonalem Maus anti-CEA sensibilisierte Polystyrolkugel (Durchmesser 6,5 mm) zugefügt und bei 37 °C während 24 Stunden inkubiert.

Anschliessend werden die Polystyrolkugeln dreimal mit je 2 bis 5 ml dest. Wasser gewaschen und dann in Teströhrchen transferiert, die je 0,250 ml Pufferlösung mit $2 \times 10^{8}$ M/l Kaninchen anti-CEA enthalten, das mit Porphyrinderivat markiert ist (Markierungsgrad 0,42). Nach einer 24-stündigen Inkubation bei 37 °C werden die Kugeln wieder dreimal mit je 2 bis 5 ml dest. Wasser gewaschen und anschliessend in Teströhrchen mit je 2 ml Schwefelsäure (0,09 N) transferiert. Nach 30 Minuten pipettiert man die Schwefelsäure-Lösung in Messküvetten und misst den Gehalt an Porphinderivat (bzw. anti-CEA) fluoreszenz-spektroskopisch (Anregungswellenlänge 433 nm ; Emissionswellenlänge 670 nm).

In Tabelle I sind die Werte einer CEA-Bestimmung aufgeführt, die mit einer Reihe von CEA-Standards von ROCHE erhalten wurden.

Tabelle I : Fluoreszenzspektroskopische Bestimmung von CEA-Standards

| Konzentration an CEA (ROCHE Standard Lösungen) | Rel. Fluoreszenzintensität |
|---|---|
| 0 ng/ml CEA | 0,275 |
| 2 ng/ml CEA | 0,750 |
| 5 ng/ml CEA | 1,325 |
| 10 ng/ml CEA | 2,400 |
| 20 ng/ml CEA | 3,400 |

In Figur I wird die relative Fluoreszenzintensität gegen die Konzentrationen an CEA in ng/ml

aufgetragen.

Beispiel 9

Mit dem gleichen Vorgehen wie in Beispiel 8 wurde eine normale humane Serumprobe analysiert, wobei ein CEA Gehalt von 1 ng/ml gefunden wurde.

**Patentansprüche**

1. Porphyrinderivate der allgemeinen Formel

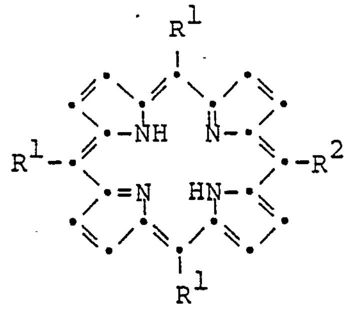

(I)

worin entweder $R^1$ die Gruppe

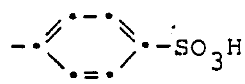

und $R^2$ die Gruppe

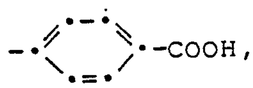

,

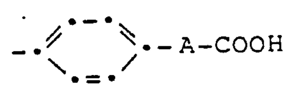

oder

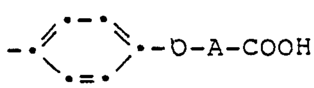

oder $R^1$ die Gruppe

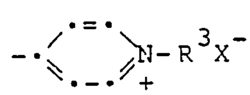

und $R^2$ die Gruppe

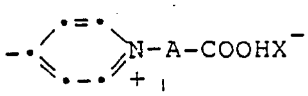

bedeuten, wobei $X^-$ ein Chlor-, Brom- oder Jodanion, ein Arylsulfonation, ein Alkylsulfonation oder ein Alkylsulfation, A($C_{1-8}$)-Alkylen, und $R^3$($C_{1-4}$)-Alkyl bedeuten, und Sulfonsäuresalze von Verbindungen der Formel I, worin $R^1$ die Gruppe

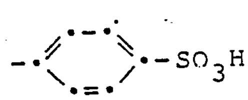

bedeutet.

2. 4-[10,15,20-Tris (4-sulfophenyl)-21H,23H-porphin-5-yl]benzoesäure.
3. [4-[10,15,20-Tris (4-sulfophenyl)-21H,23H-porphin-5-yl]phenoxy]essigsäure.
4. 6-[4-[10,15,20-Tris (4-sulfophenyl)-21H,23H-porphin-5-yl]phenyl]hexancarbonsäure.

5. 1-(Carboxymethyl)-1′,1″,1‴-trimethyl-4,4′,4″,4‴-(21H,23H-porphin-5,10,15,20-tetrayl)tetrakispyridinium tetrajodid.

6. 1-(2-Carboxyäthyl)-1′,1″,1‴-trimethyl-4,4′,4″,4‴-(21H,23H-porphin-5,10,15,20-tetrayl)tetrakispyridinium bromid trijodid.

7. 1-(5-Carboxypentyl)-1′,1″,1‴-trimethyl-4,4′,4″,4‴-(21H,23H-porphin-5,10,15,20-tetrayl)tetrakispyridinium bromid trijodid.

8. Verbindungen der allgemeinen Formel

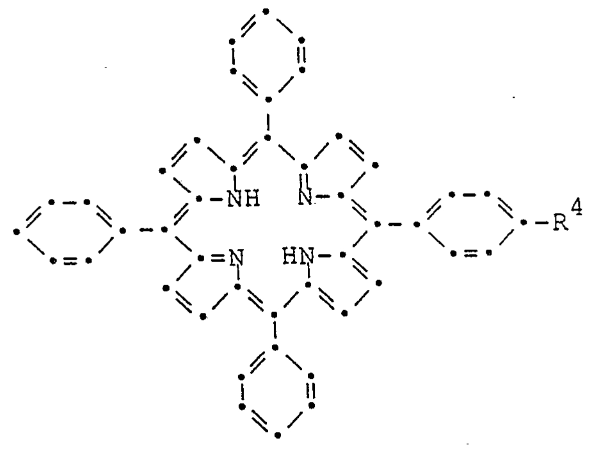

(II)

worin $R^4$ die Gruppe —COOH, —A—COOH oder —O—A—COOH und $A(C_{1-8})$-Alkylen bedeuten.

9. Verbindungen der allgemeinen Formel

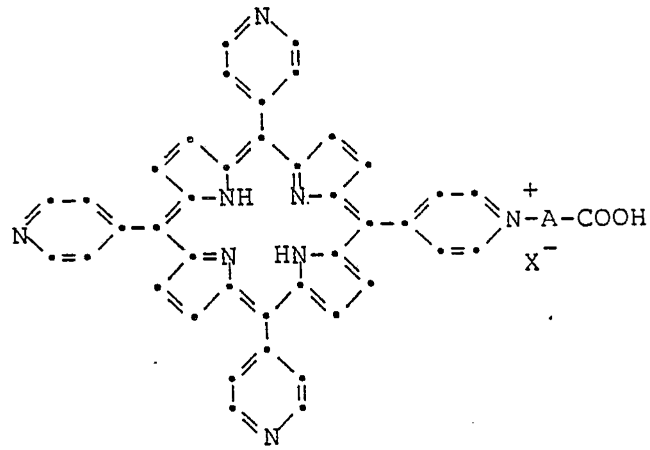

(III)

worin $X^-$ ein Chlor-, Brom- oder Jodanion, ein Arylsulfonation, ein Alkylsulfonation oder ein Alkylsulfation und $A(C_{1-8})$-Alkylen bedeuten.

10. Verbindungen der allgemeinen Formel I als Marker in Fluoreszenz-Immuno-Assays.

11. Verfahren zur Herstellung von Verbindungen gemäss einem der Ansprüche 1 bis 7, dadurch gekennzeichnet, dass man

a) eine Verbindung der allgemeinen Formel

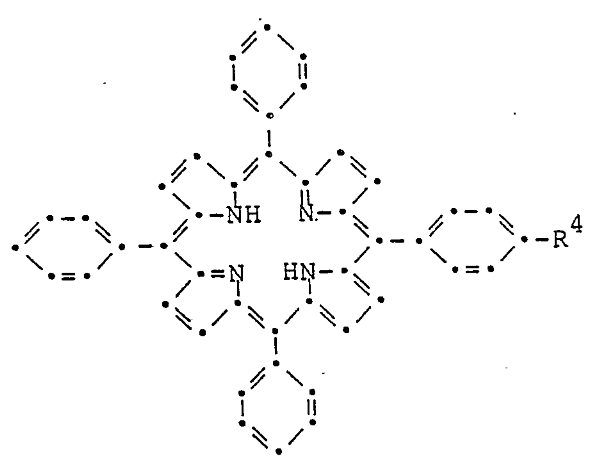

(II)

worin $R^4$ die Gruppe —COOH, —A—COOH oder —O—A—COOH und $A(C_{1-8})$-Alkylen bedeuten, mit einem die Gruppe $—SO_3H$ liefernden Mittel umsetzt, oder

b) eine Verbindung der allgemeinen Formel

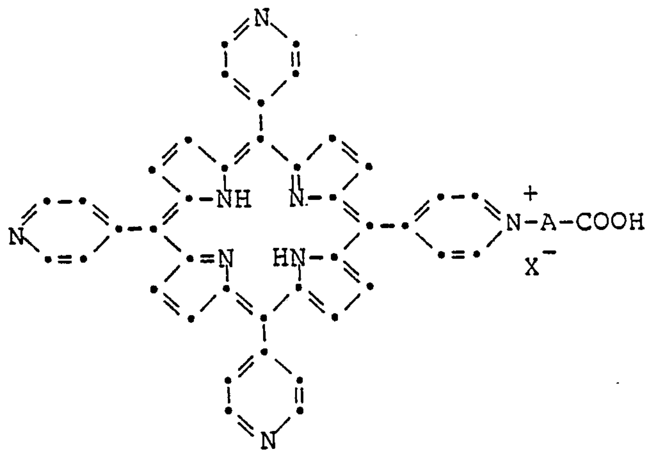

(III)

worin $A(C_{1-8})$-Alkylen und $X^-$ ein Chlor-, Brom- oder Jodanion, ein Arylsulfonation, ein Alkylsulfonation oder ein Alkylsulfation bedeuten, mit einem einen $(C_{1-4})$-Alkylrest liefernden Mittel behandelt und erwünschtenfalls

c) eine nach Verfahren a) erhaltene Verbindung in ein Sulfonsäuresalz überführt.

12. Verwendung der Verbindungen der allgemeinen Formel I als Marker in Fluoreszenz-Immuno-Assays.

13. Verwendung der Verbindungen der allgemeinen Formel I als Marker in Fluoreszenz-Immuno-Assays mit zeitaufgelöster Fluoreszenzmessung.

14. Verwendung der Verbindungen gemäss allgemeiner Formel I nach Patentanspruch 12 oder 13 zur Bestimmung von CEA.

**Claims**

1. Porphyrin derivatives of the general formula

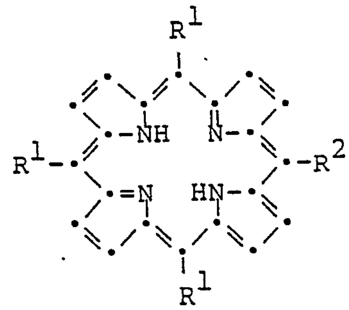

(I)

wherein either $R^1$ signifies the group

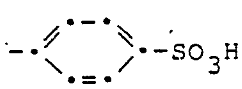

and $R^2$ signifies the group

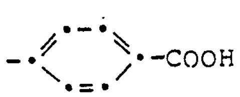

,

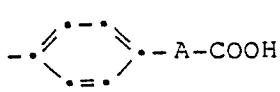

or

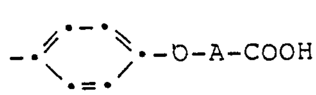

or $R^1$ signifies the group

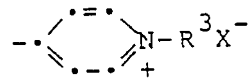

and $R^2$ signifies the group

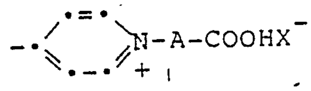

whereby $X^-$ signifies a chlorine, bromine or iodine anion, an arylsulphonate ion, and alkylsulphonate ion or an alkyl sulphate ion, A signifies $(C_{1-8})$-alkylene and $R^3$ signifies $(C_{1-4})$-alkyl, and sulphonic acid salts of compounds of formula I in which $R^1$ signifies the group

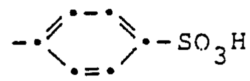

2. 4-[10,15,20-Tris(4-sulphophenyl)-21H,23H-porphin-5-yl]benzoic acid.
3. [4-[10,15,20-Tris(4-sulphophenyl)-21H,23H-porphin-5-yl]phenoxy]acetic acid.
4. 6-[4-[10,15,20-Tris(4-sulphophenyl)-21H,23H-porphin-5-yl]phenyl]hexanecarboxylic acid.
5. 1-(Carboxymethyl)-1′,1″,1‴-trimethyl-4,4′,4″,4‴-(21H,23H-porphin-5,10,15,20-tetrayl)tetrakis-pyridinium tetraiodide.
6. 1-(2-Carboxyethyl)-1′,1″,1‴-trimethyl-4,4′,4″,4‴-(21H,23H-porphin-5,10,15,20-tetrayl)tetrakis-pyridinium bromide triiodide.
7. 1-(5-Carboxypentyl)-1′,1″,1‴-trimethyl-4,4′,4″,4‴-(21H,23H-porphin-5,10,15,20-tetrayl)tetrakis-pyridinium bromide triiodide.
8. Compounds of the general formula

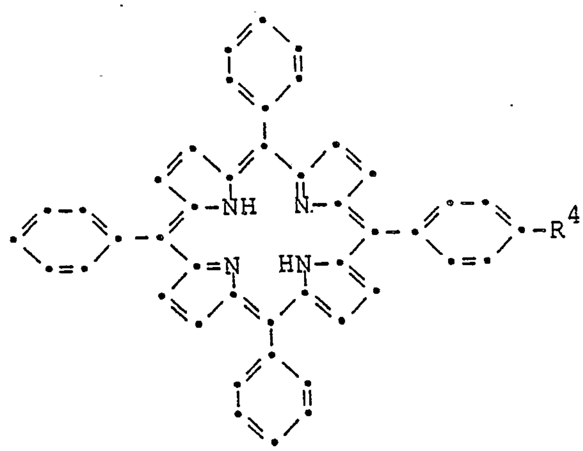

(II)

wherein $R^4$ signifies the group —COOH, —A—COOH or —O—A—COOH and A signifies $(C_{1-8})$-alkylene.

9. Compounds of the general formula

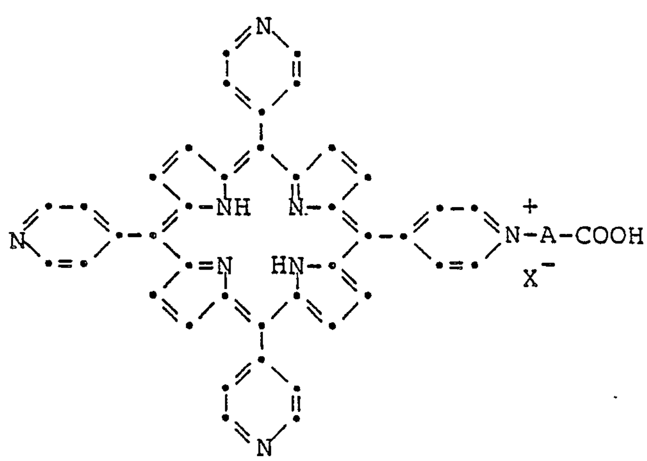

(III)

wherein $X^-$ signifies a chlorine, bromine or iodine anion, an arylsulphonate ion, an alkylsulphonate ion or an alkyl sulphate ion and A signifies $(C_{1-8})$-alkylene.

10. Compounds of general formula I as labels in fluorescence immuno-assays.

11. A process for the manufacture of compounds in accordance with any one of claims 1 to 7, characterized by

a) reacting a compound of the general formula

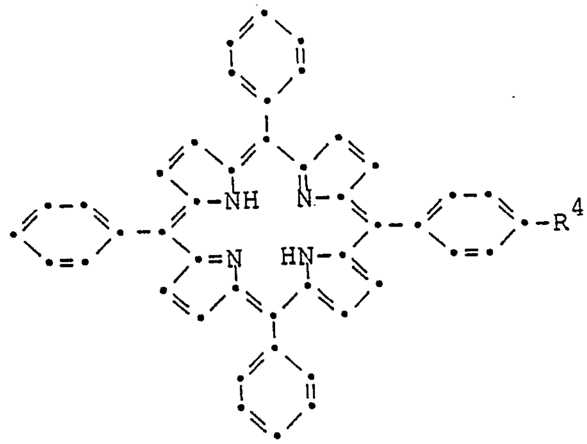

(II)

wherein $R^4$ signifies the group —COOH, —A—COOH or —O—A—COOH and A signifies $(C_{1\text{-}8})$-alkylene, with an agent yielding the group $—SO_3H$, or

b) treating a compound of the general formula

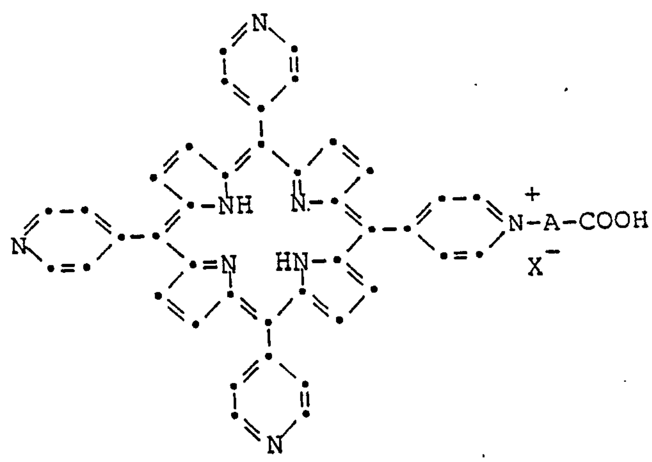

(III)

wherein A signifies $(C_{1\text{-}8})$-alkylene and $X^-$ signifies a chlorine, bromine or iodine anion, an arylsulphonate ion, an alkylsulphonate ion or an alkyl sulphate ion, with an agent yielding a $(C_{1\text{-}4})$-alkyl residue and, if desired,

c) converting a compound obtained according to process a) into a sulphonic acid salt.

12. The use of the compounds of general formula I as labels in fluorescence immuno-assays.

13. The use of the compounds of general formula I as labels in fluorescence immuno-assays with a time-resolved fluorescence measurement.

14. The use of the compounds in accordance with general formula I according to Patent Claim 12 or 13 for the determination of CEA.

**Revendications**

1. Dérivés de porphyrines de formule générale

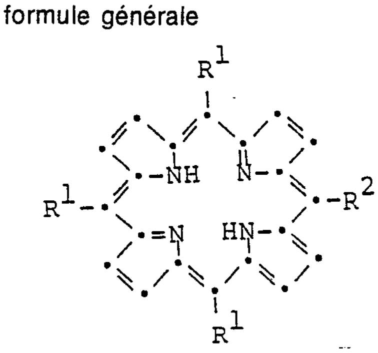

(I)

dans laquelle $R^1$ représente le groupe

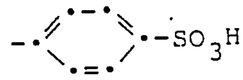

et $R^2$ représente le groupe

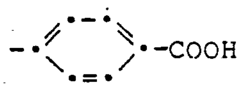 , 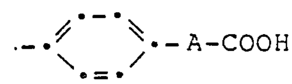

ou

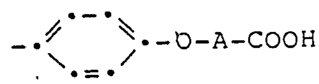

ou bien $R^1$ représente le groupe

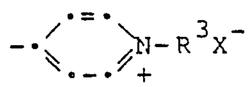

et $R^2$ le groupe

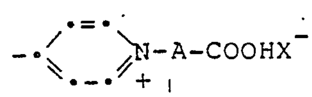

dans lesquels $X^-$ représente un anion chlore, brome ou iode, un ion arylsulfonate, alkylsulfonate ou alkylsulfate, A représente un groupe alkylène en $C_1$-$C_8$ et $R^3$ un groupe alkyle en $C_1$-$C_4$, et les sels d'acides sulfoniques des composés de formule I dans lesquels $R^1$ représente le groupe

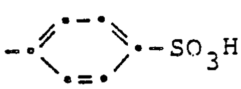

2. L'acide 4-[10,15,20-tris-(4-sulfophényl)-21H,23H-porphine-5-yl]-benzoïque.
3. L'acide [4-[10,15,20-tris-(4-sulfophényl)-21H,23H-porphine-5-yl]-phénoxy]acétique.
4. L'acide 6-[4-[10,15,20-tris-(4-sulfophényl)-21H,23H-porphine-5-yl]-phényl]-hexane-carboxylique.
5. Le tétraiodure de 1-(carboxyméthyl)-1',1'',1'''-triméthyl-4,4',4'',4'''-(21H,23H-porphine-5,10,15,20-tétrayl)-tétrakis-pyridinium..
6. Le bromure-triodure de 1-(2-carboxyéthyl)-1',1'',1'''-triméthyl-4,4',4'',4''(21H,23H-porphine-5,10,15,20-tétrayl)-tétrakis-pyridinium.
7. Le bromure-triodure de 1-(5-carboxypentyl)-1',1'',1'''-triméthyl-4,4',4'',4'''-(21H,23H-porphine-5,10,15,20-tétrayl)-tétrakis-pyridinium.
8. Composés de formule générale

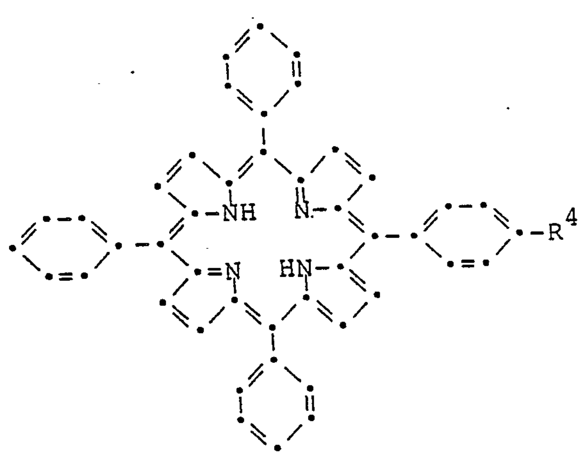

(II)

dans laquelle $R^4$ représente un groupe —COOH, —A—COOH ou —O—A—COOH et A représente un groupe alkylène en $C_1$-$C_8$.

9. Composés de formule générale

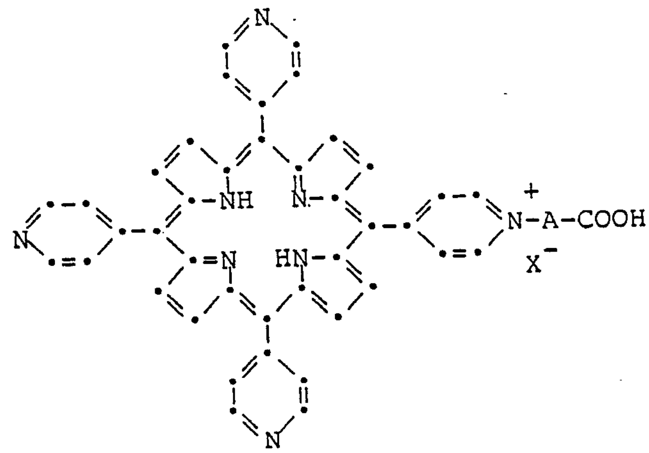

(III)

dans laquelle $X^-$ représente un anion chlore, brome ou iode, un ion arylsulfonate, alkylsulfonate ou alkylsulfate et A représente un groupe alkylène en $C_1$-$C_8$.

10. Les composés de formule générale I en tant que marqueurs dans des immunotests à fluorescence.

11. Procédé de préparation des composés selon l'une des revendications 1 à 7, caractérisé en ce que :

a) on fait réagir un composé de formule générale

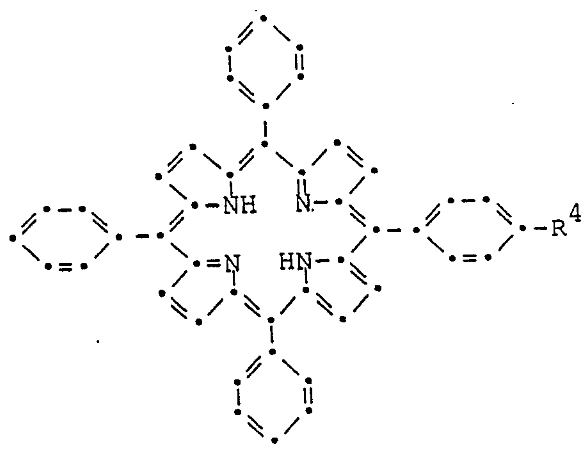

(II)

dans laquelle $R^4$ représente un groupe —COOH, —A—COOH ou —O—A—COOH et A représente un groupe alkylène en $C_1$-$C_8$, avec un réactif apportant le groupe $—SO_3H$, ou bien

b) on traite un composé de formule générale

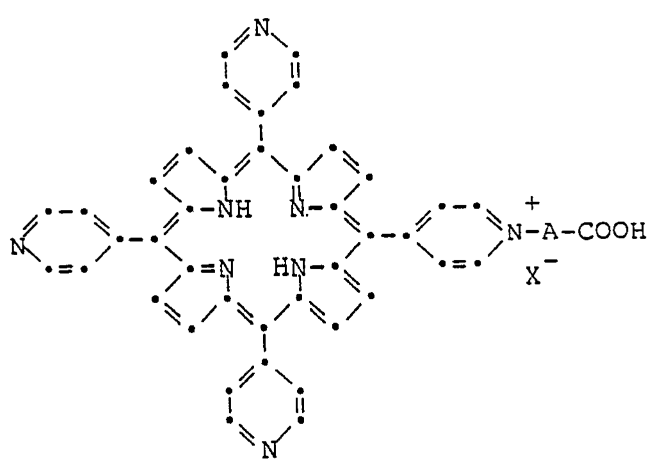

(III)

dans laquelle A représente un groupe alkylène en $C_1$-$C_8$ et $X^-$ représente un anion chlore, brome ou

iode, un ion arylsulfonate, alkylsulfonate ou alkylsulfate, par un réactif apportant un groupe alkyle en $C_1$-$C_4$, et si on le désire,

c) on convertit un composé obtenu par le procédé a) en un sel d'acide sulfonique.

12. Utilisation des composés de formule générale I en tant que marqueurs dans des immunotests à fluorescence.

13. Utilisation des composés de formule générale I en tant que marqueurs dans des immunotests à fluorescence avec mesure d'une fluorescence disparaissant dans le cours du temps.

14. Utilisation des composés de formule générale I selon la revendication 12 ou 13, pour le dosage du CEA.

FIG. 1

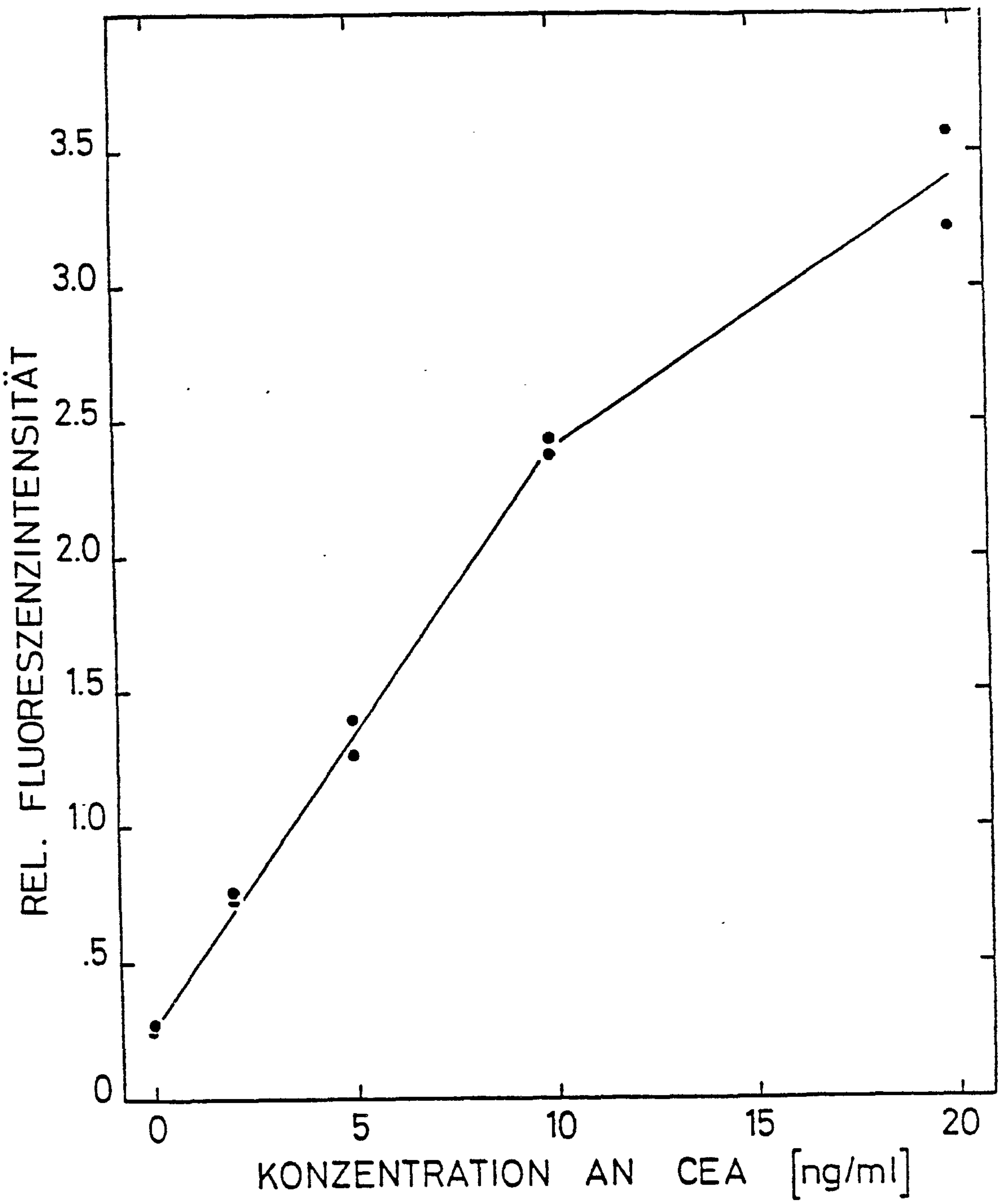
REL. FLUORESZENZINTENSITÄT
3.5
3.0
2.5
2.0
1.5
1.0
.5
0
0
5
10
15
20
KONZENTRATION AN CEA [ng/ml]